(19) Europäisches Patentamt
European Patent Office
Office européen des brevets

(11) Veröffentlichungsnummer: **0 064 250**
**B1**

(12) # EUROPÄISCHE PATENTSCHRIFT

(45) Veröffentlichungstag der Patentschrift: **14.08.85**

(51) Int. Cl.⁴: **C 09 B 62/06**, C 09 B 62/505, D 06 P 3/66, D 06 P 3/10

(21) Anmeldenummer: **82103500.3**

(22) Anmeldetag: **24.04.82**

(54) **Anthrachinonverbindungen, Verfahren zu ihrer Herstellung und ihre Verwendung als Farbstoffe.**

(30) Priorität: **02.05.81 DE 3117482**

(43) Veröffentlichungstag der Anmeldung: **10.11.82 Patentblatt 82/45**

(45) Bekanntmachung des Hinweises auf die Patenterteilung: **14.08.85 Patentblatt 85/33**

(84) Benannte Vertragsstaaten: **CH DE FR GB IT LI**

(56) Entgegenhaltungen:
**DE - B - 1 265 698**
**FR - A - 1 546 109**
**FR - A - 2 437 426**

**CHEMICAL ABSTRACTS, Band 96, Nr. 6, February 1982, Seite 90, Nr. 36871f, Columbus, Ohio, USA**
**CHEMICAL ABSTRACTS, Band 84, Nr.18, 3. Mai 1976, Seite 83, Nr. 123403a, Columbus, Ohio, USA**

***Die Akte enthält technische Angaben, die nach dem Eingang der Anmeldung eingereicht wurden und die nicht in dieser Patentschrift enthalten sind.***

(73) Patentinhaber: **HOECHST AKTIENGESELLSCHAFT, Postfach 80 03 20, D-6230 Frankfurt am Main 80 (DE)**

(72) Erfinder: **Meininger, Fritz, Dr., Loreleistrasse 7, D-6230 Frankfurt am Main 80 (DE)**
Erfinder: **Otten, Joachim, Dr., deceased (DE)**

**EP 0 064 250 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung liegt auf dem technischen Gebiet der faserreaktiven Farbstoffe.

Aus der französischen Patentschrift Nr. 1 546 109 ergibt sich ein Anthrachinonfarbstoff, der in 4-Stellung des Anthrachinons einen 3-Amino-2,4,6-trimethyl-2-sulfophenylamino-Rest gebunden enthält, an dessen freie Aminogruppe wiederum ein 2-Chlor-4-(N-β-sulfatoethyl-sulfamoyl)-phenylamino-s-triazinyl-Rest gebunden ist; dieser Farbstoff zeigt sowohl in einem Einphasen-Druckverfahren als auch im Ausziehverfahren keine ausreichend guten Fixiergrade.

Aus der französischen Patentanmeldungs-Veröffentlichung Nr. 2 437 426 sind Anthrachinonfarbstoffe bekannt, die einen 2-Fluor-4-(β-sulfatoethylsulfonyl)-phenylamino-s-triazinyl-Rest enthalten, der ebenso über eine Aminogruppe an einen methylsubstituierten Sulfophenylaminorest in 4-Stellung des 1-Amino-2-sulfo-anthrachinons gebunden ist, wobei dieser Fluortriazinylamino-Rest entweder direkt an den Benzolkern des Methyl-sulfophenylamino-Restes oder direkt an eine von dessen Methylgruppen gebunden ist. Insbesondere der letztgenannte Farbstofftyp zeigt einen sehr schlechten Fixiergrad im Ausziehverfahren und in einem Einphasen-Druckverfahren und darüberhinaus keine Druckpastenstabilität von natriumcarbonathaltigen Druckpasten.

Es wurden neue, wertvolle, wasserlösliche Anthrachinonverbindungen entsprechend der allgemeinen Formel (1)

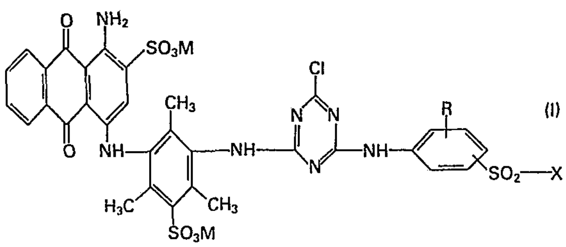

(I)

gefunden, in welcher

M für ein Wasserstoffatom oder das Äquivalent eines Metalls, wie insbesondere eines Alkali- oder Erdalkalimetalls, wie beispielsweise des Natriums, Kaliums, Lithiums und Calciums, steht,

R ein Wasserstoffatom, wie Methyl-, Ethyl-, Methoxy-, Ethoxy- oder Carboxygruppe oder ein Halogenatom, wie ein Brom- und insbesondere Chloratom, ist und

X die Vinylgruppe oder eine β-Sulfatoethyl-Gruppe (entsprechend der Formel $-CH_2-CH_2-OSO_3M$ mit M der obengenannten Bedeutung) oder eine β-Thiosulfatoethyl-Gruppe (entsprechend der Formel $-CH_2-CH_2-S-SO_3M$ mit M der obengenannten Bedeutung) oder die β-Chlorethyl-Gruppe bedeutet.

Die neuen Anthrachinonverbindungen können sowohl in saurer Form als auch in Form ihrer Salze vorliegen. Bevorzugt sind sie in Form der Salze, insbesondere der Alkali- und Erdalkalimetallsalze, und finden auch bevorzugt in Form dieser Salze Verwendung zum Färben und Bedrucken von hydroxy- oder carbonamidgruppenhaltigen Materialien, insbesondere Fasermaterialien.

Die vorliegende Erfindung betrifft weiterhin Verfahren zur Herstellung der obengenannten und definierten Anthrachinonverbindung der allgemeinen Formel (1). Diese sind dadurch gekennzeichnet, dass man eine Verbindung der allgemeinen Formel (2)

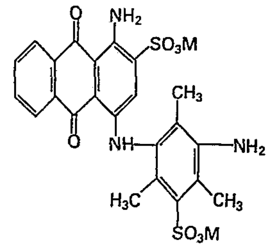

(2)

in welcher M die obengenannte Bedeutung hat, mit Trichlortriazin (Cyanurchlorid) in äquimolaren Mengen zur Dichlortriazinylverbindung der allgemeinen Formel (3)

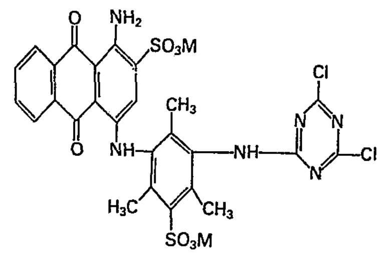

(3)

mit M der obengenannten Bedeutung umsetzt und diese mit einem aromatischen Amin der allgemeinen Formel (4)

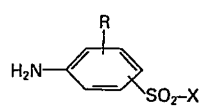

(4)

in welcher R und X die obengenannten Bedeutungen haben, unter Bildung der Verbindung entsprechend der allgemeinen Formel (1) kondensiert oder dass man eine Anthrachinonverbindung der oben angegebenen und definierten allgemeinen Formel (2) mit einer Dichlortriazinylverbindung der allgemeinen Formel (5)

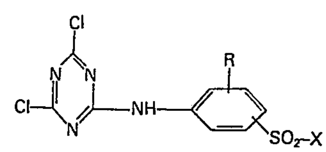

(5)

mit R und X der obengenannten Bedeutung umsetzt.

Die Kondensation der Anthrachinonverbindung der allgemeinen Formel (2) mit Cyanurchlorid wird im allgemeinen im wässrigen Medium oder in einem wässrig-organischen Lösemittel bei einer Temperatur zwischen −5°C und +30°C vorzugsweise zwischen 0°C und +10°C, und bei einem pH-Wert zwischen 3 und 9, vorzugsweise zwischen 5 und 8, durchgeführt: die Kondensationsreaktion der Dichlortriazinylverbindung der

allgemeinen Formel (3) mit dem aromatischen Amin der allgemeinen Formel (4) kann bei einer Temperatur zwischen 0°C und 70°C, vorzugsweise 0°C und 50°C, erfolgen.

Die Umsetzung der Anthrachinonverbindung der allgemeinen Formel (2) mit einer Dichlortriazinylverbindung der allgemeinen Formel (5) wird in der Regel im wässrigen Medium, gegebenenfalls unter Mitverwendung eines organischen Lösemittels, bei einer Temperatur zwischen 0°C und 60°C, vorzugsweise zwischen 0°C und 50°C, und bei einem pH-Wert zwischen 3 und 9, vorzugsweise zwischen 5 und 8, durchgeführt.

Die bei den Kondensationsreaktionen freiwerdende Chlorwasserstoffsäure wird mittels säurebindender Mittel, wie beispielsweise Natrium- oder Kaliumhydroxid, Natrium- oder Kaliumcarbonat, Natrium- oder Kaliumbicarbonat, Natriumacetat oder einem basischen Natriumphosphat gebunden.

Die Abscheidung der erfindungsgemäss hergestellten Verbindungen der allgemeinen Formel (1) aus der Syntheselösung kann nach allgemein bekannten Methoden erfolgen, so beispielsweise entweder durch Ausfällen aus dem Reaktionsmedium mittels Elektrolyten, wie beispielsweise Natriumchlorid oder Kaliumchlorid, oder durch Eindampfen der Reaktionslösung, beispielsweise Sprühtrocknung, wobei dieser Reaktionslösung eine Pufferstubstanz zugefügt werden kann.

Die erfindungsgemässen Verbindungen der allgemeinen Formel (1) haben faserreaktive Eigenschaften und besitzen sehr gute Farbstoffeigenschaften. Sie können deshalb zum Färben (einschliesslich Bedrucken) von hydroxygruppenhaltigen und/oder carbonamidgruppenhaltigen Materialien verwendet werden. Auch können die bei der Synthese der erfindungsgemässen Verbindungen anfallenden Lösungen, gegebenenfalls nach Zusatz einer Puffersubstanz, gegebenenfalls auch nach Konzentrierung, direkt als Flüssigpräparation der färberischen Verwendung zugeführt werden.

Gegenstand der vorliegenden Erfindung ist deshalb auch die Verwendung der erfindungsgemässen Verbindungen der allgemeinen Formel (1) zum Färben (einschliesslich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, Wickelkörpern und Geweben.

Hydroxygruppenhaltige Materialien sind solche natürlichen oder synthetischen Ursprungs, wie beispielsweise Cellulosefasermaterialien oder deren Regeneratprodukte und Polyvinylalkohole. Cellulosefasermaterialien sind vorzugsweise Baumwolle, aber auch andere Pflanzenfasern, wie Leinen, Hanf, Jute und Ramifasern; regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form von Fasern, beispielsweise Wolle und andere Tierhaare, Seide, Leder, Polyamid-6,6, Polyamid-6, Polyamid-11 und Polyamid-4.

Die erfindungsgemässen Verbindungen der allgemeinen Formel (1) lassen sich auf den genannten Substraten, insbesondere auf den genannten Fasermaterialien, nach den für wasserlösliche, insbesondere nach den für wasserlösliche faserreaktive Farbstoffe bekannten Anwendungstechniken applizieren und fixieren.

So erhält man mit ihnen auf Cellulosefasern nach den Ausziehverfahren aus langer Flotte unter Verwendung von verschiedensten säurebindenden Mitteln und gegebenenfalls neutralen Salzen, wie Natriumchlorid oder Natriumsulfat, sehr gute Farbausbeuten sowie einen ausgezeichneten Farbaufbau. Man färbt bevorzugt in wässrigem Bad bei Temperaturen zwischen 60 und 105°C, gegebenenfalls bei Temperaturen bis zu 120°C unter Druck und gegebenenfalls in Gegenwart von üblichen Färbereihilfsmitteln. Man kann dabei so vorgehen, dass man das Material in das warme Bad einbringt und dieses allmählich auf die gewünschte Färbetemperatur erwärmt und den Färbeprozess bei dieser Temperatur zu Ende führt. Die das Ausziehen des Farbstoffes beschleunigenden Neutralsalze können dem Bade gewünschtenfalls auch erst nach Erreichen der eigentlichen Färbetemperatur zugesetzt werden.

Nach dem Klotzverfahren werden auf Cellulosefasern ebenfalls ausgezeichnete Farbausbeuten und ein sehr guter Farbaufbau erhalten, wobei durch Verweilen bei Raumtemperatur oder erhöhter Temperatur, beispielsweise bis zu etwa 60° C, durch Dämpfen oder mit Trockenhitze in üblicher Weise fixiert werden kann.

Ebenfalls nach den üblichen Druckverfahren für Cellulosefasern, die einphasig beispielsweise durch Bedrucken mit einer Natriumbicarbonat oder ein anderes säurebindendes Mittel enthaltenden Druckpaste und anschliessendes Dämpfen bei 100 bis 103° C oder zweiphasig beispielsweise durch Bedrucken mit neutraler oder schwach saurer Druckfarbe und anschliessendes Fixieren durch Hindurchführen des bedruckten Materials durch ein heisses elektrolythaltiges alkalisches Bad oder durch Überklotzen des bedruckten Materials mit einer alkalischen elektrolythaltigen Klotzflotte und anschliessendes Verweilen oder Dämpfen oder Behandlung mit Trockenhitze dieses überklotzten Materials durchgeführt werden können, erhält man farbstarke Drucke mit gutem Stand der Konturen und einem klaren Weissfond. Der Ausfall der Drucke ist von wechselnden Fixierbedingungen nur wenig abhängig.

Bei der Fixierung mittels Trockenhitze nach den üblichen Thermofixierverfahren verwendet man Heissluft von 120 bis 200° C. Neben dem üblichen Wasserdampf von 101 bis 103° C kann auch überhitzter Dampf und Druckdampf von Temperaturen bis zu 160° C eingesetzt werden.

Die säurebindenden und die Fixierung der Verbindung der Formel (1) auf den Cellulosefasern bewirkenden Mittel sind beispielsweise wasserlösliche basische Salze der Alkalimetalle und

ebenfalls Erdalkalimetalle von anorganischen oder organischen Säuren oder Verbindungen, die in der Hitze Alkali freisetzen. Insbesondere sind die Alkalimetallhydroxide und Alkalimetallsalze von schwachen bis mittelstarken anorganischen oder organischen Säuren zu nennen, wobei von den Alkaliverbindungen vorzugsweise die Natrium- und Kaliumverbindungen gemeint sind. Solche säurebindenden Mittel sind beispielsweise Natriumhydroxid, Kaliumhydroxid, Natriumcarbonat, Natriumbicarbonat, Kaliumcarbonat, Natriumformiat, Natriumdihydrogenphosphat, Dinatriumhydrogenphosphat, Natriumtrichloracetat, Wasserglas oder Trinatriumphosphat.

Durch die Behandlung der erfindungsgemässen Verbindungen mit den säurebindenden Mitteln, gegebenenfalls unter Wärmeeinwirkung, werden die erfindungsgemässen Verbindungen (Farbstoffe) chemisch an die Cellulosefaser gebunden; sie zeichnen sich auf diesen Fasermaterialien bei Anwendung in den Färbe- und Druckverfahren durch eine hohe Fixierausbeute aus. Die Cellulosefärbungen zeigen nach der üblichen Nachbehandlung durch Spülen zur Entfernung von nicht fixierten Farbstoffanteilen ausgezeichnete Nassechtheiten, zumal sich nicht fixierte Farbstoffanteile leicht wegen ihrer guten Kaltwasserlöslichkeit auswaschen lassen.

Die Färbungen auf Polyurethan- und Polyamidfasern werden üblicherweise aus saurem Milieu ausgeführt. So kann man beispielsweise dem Färbebad Essigsäure und/oder Ammoniumsulfat oder Essigsäure und Ammoniumacetat oder Natriumacetat zufügen, um den gewünschten pH-Wert zu erhalten. Um eine brauchbare Egalität der Färbung zu erreichen, empfiehlt sich ein Zusatz an üblichen Egalisierhilfsmitteln, wie beispielsweise auf Basis eines Umsetzungsproduktes von Cyanurchlorid mit der 3fach molaren Menge einer Aminobenzolsulfonsäure und/oder einer Aminonaphthalinsulfonsäure oder auf Basis eines Umsetzungsproduktes von beispielsweise Stearylamin mit Ethylenoxid. In der Regel wird das zu färbende Material bei einer Temperatur von etwa 40° C in das Bad eingebracht, dort einige Zeit darin bewegt, das Färbebad dann auf den gewünschten schwach sauren, vorzugsweise schwach essigsauren, pH-Wert nachgestellt und die eigentliche Färbung bei einer Temperatur zwischen 60 und 98° C durchgeführt. Die Färbungen können aber auch bei Siedetemperatur oder bei Temperaturen bis zu 120° C (unter Druck) ausgeführt werden.

Die mit den erfindungsgemässen Verbindungen der allgemeinen Formel (1) hergestellten Färbungen und Drucke zeichnen sich durch sehr klare Nuancen aus. Insbesondere besitzen die Färbungen und Drucke auf Cellulosefasermaterialien eine gute Lichtechtheit und sehr gute Nassechtheiten, wie Wasch-, Walk-, Wasser-, Seewasser-, Überfärbe- und Schweissechtheiten, desweiteren eine gute Plissierechtheit, Bügelechtheit und Reibechtheit.

Von besonderer Bedeutung ist die erfindungsgemässe Verwendung der Verbindungen der allgemeinen Formel (1) auch für das faserreaktive Färben von Wolle. Insbesondere lässt sich auch filzfrei oder filzarm ausgerüstete Wolle (vgl. bspw. H. Rath, Lehrbuch der Textilchemie, Springer-Verlag, 3. Auflage (1972), S. 295–299, insbesondere die Ausrüstung nach dem sogenannten Hercosett-Verfahren (S. 298); J. Soc. Dyers and Colourists 1972, 93–99, und 1975, 33–44) mit sehr guten Echtheitseigenschaften färben.

Das Verfahren des Färbens auf Wolle erfolgt hierbei in üblicher und bekannter Färbeweise, indem die faserreaktive Verbindung der allgemeinen Formel (1) bevorzugt zunächst aus saurem Färbebad mit einem pH von etwa 3,5 bis 5,5 unter Kontrolle des pH-Wertes dem Ausziehprozess unterworfen wird und gegen Ende der Färbezeit der pH-Wert in den neutralen und gegebenenfalls schwach alkalischen Bereich bis zu einem pH-Wert von 8,5 verschoben wird, um besonders bei einer erwünschten Erzielung von hohen Farbtiefen die volle reaktive Bindung zwischen diesem Farbstoff der Formel (1) und der Faser herbeizuführen. Gleichzeitig wird der nicht reaktiv gebundene Farbstoffanteil abgelöst.

Die hier beschriebene Verfahrensweise gilt auch zur Herstellung von Färbungen auf Fasermaterialien aus anderen natürlichen Polyamiden oder aus synthetischen Polyamiden und Polyurethanen. Die Färbungen werden bei Temperaturen von 60 bis 100°C durchgeführt, jedoch können sie auch in geschlossenen Färbeapparaturen bei Temperaturen bis zu 106°C erfolgen. Da die Wasserlöslichkeit der Verbindungen der allgemeinen Formel (1) sehr gut ist, lassen sie sich auch mit Vorteil bei üblichen kontinuierlichen Färbeverfahren einsetzen. Die Farbstärke der erfindungsgemässen Verbindungen der allgemeinen Formel (1) ist sehr hoch. Sie liefern auf den Fasermaterialien, insbesondere beim reaktiven Färben von Wolle, sehr klare, rotstichig blaue Färbungen. Bei Anwendung von Färbetemperaturen von 100 bis 106°C ist eine hohe Baderschöpfung festzustellen.

Bei den mit den erfindungsgemässen Verbindungen der allgemeinen Formel (1) erhältlichen Färbungen kann auf eine ansonsten übliche ammoniakalische Nachbehandlung der gefärbten Ware verzichtet werden. Im Vergleich zu konstitutionell ähnlichen, bekannten Farbstoffen zeigen sie in überraschender Weise einen sehr guten Farbaufbau, wobei die brillante Nuance auch in tiefen Tönen erhalten bleibt. Darüber hinaus zeigen sie eine gute Kombinierbarkeit mit anderen faserreaktiven Wollfarbstoffen, die ein überraschend egales Färben der Faser ermöglichen. Ebenso lässt sich Material aus Wollfasern unterschiedlicher Provenienz mit den erfindungsgemässen Verbindungen egal färben. Zur Verbesserung des Egalisierverhaltens kann gegebenenfalls ein übliches Egalisierhilfsmittel, wie beispielsweise N-Methyltaurin, zugesetzt werden.

Unter Verwendung der üblichen faseraffinen Färbehilfsmittel ergeben die erfindungsgemässen Verbindungen auch auf filzfrei oder filzarm ausgerüsteter Wolle egale Färbungen. Bei hellen bis mittleren Farbtiefen ist auch ohne ammoniakalische Nachbehandlung ein sehr gutes Nassechtheitsniveau zu erzielen, wobei jedoch gegebenenfalls eine ammoniakalische Nachbehandlung bevorzugt werden kann. Neben der hohen Lichtechtheit dieser Wollfärbungen sind als sehr gute Nassechtheitseigenschaften insbesondere die ausgezeichnete alkalische Schweissechtheit und sehr gute Waschechtheit bei 60°C, auch von Färbungen in hohen Farbtiefen, zu nennen.

Von den erfindungsgemässen Verbindungen können insbesondere die Verbindungen der allgemeinen Formel (6)

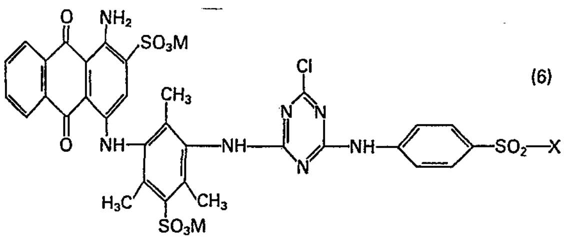

(6)

hervorgehoben werden, in welcher X für die Vinylgruppe, eine β-Thiosulfatoethyl- oder eine β-Sulfatoethylgruppe steht und M die obengenannte Bedeutung hat, vorzugsweise Natrium oder Kalium, bedeutet.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wir Kilogramm zu Liter.

Beispiel 1

Eine Lösung mit einem pH-Wert von 7,5 bis 8,0 von 53,1 Teilen 1-Amino-4-(3'-amino-2',4',6'-trimethyl-5'-sulfophenylamino)-anthrachinon-2-sulfonsäure in etwa 420 Teilen Wasser lässt man zu einer Suspension von 19 Teilen Cyanurchlorid in 150 Teilen Eiswasser zutropfen. Die Temperatur des Reaktionsgemisches wird durch Kühlung auf 0 bis 5°C und der pH-Wert gleichzeitig durch Zutropfen einer Natriumcarbonatlösung auf 6 bis 7 gehalten. Bei dieser Temperatur und diesem pH-Wert wird noch 2 Stunden nachgerührt, bis die Kondensation beendet ist. Zu diesem Ansatz wird dann die neutrale Lösung von 32 Teilen 4-β-Thiosulfatoethylsulfonyl-anilin in etwa 150 Teilen Wasser gegeben. Das Reaktionsgemisch wird dann auf 60°C erwärmt und bei dieser Temperatur 2 Stunden gerührt, wobei der pH-Wert durch Zutropfen einer wässrigen Natriumcarbonatlösung auf 6,5 bis 7,0 gehalten wird. Anschliessend giesst man 5 Teile Dinatriumphosphat hinzu, und die synthetisierte Verbindung wird durch Sprühtrocknung isoliert. Es werden etwa 142 Teile eines schwarzblauen, elektrolythaltigen Pulvers erhalten, das das Natriumsalz der Verbindung der Formel

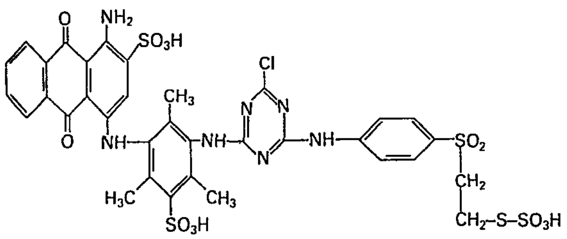

enthält. Diese Anthrachinonverbindung eignet sich hervorragend als Farbstoff. Sie kann nach den in der Technik üblichen Applikations- und Fixiermethoden für faserreaktive Farbstoffe auf den in der Beschreibung erwähnten Fasermaterialien aufgebracht und fixiert werden. Infolge ihrer faserreaktiven Eigenschaften werden klare, blaue Färbungen und Drucke mit rotstichiger Nuance, beispielsweise auf Baumwolle, erhalten, die sehr licht- und waschecht sind.

Beispiel 2

Es wird gemäss Beispiel 1 verfahren, jedoch werden in der zweiten Kondensationsstufe anstelle von 4-β-Thiosulfatoethylsulfonyl-anilin 30 Teile 4-β-Sulfatoethylsulfonyl-anilin eingesetzt. Nach Beendigung der Umsetzung wird die Reaktionslösung auf Raumtemperatur abgekühlt und die synthetisierte Verbindung mit 80 Teilen Kaliumchlorid ausgefällt, abgesaugt, mit 20%iger wässriger Kaliumchloridlösung gewaschen und unter reduziertem Druck bei 60°C getrocknet und gemahlen. Man erhält etwa 151 Teile eines schwarzblauen, elektrolythaltigen Pulvers, das das Natrium-/Kaliumsalz der Verbindung der Formel

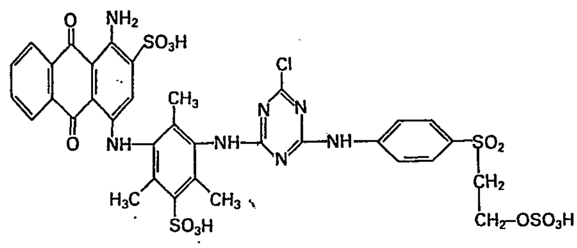

enthält. Diese Anthrachinonverbindung eignet sich ausgezeichnet als Farbstoff und liefert beispielsweise aus wässrig-alkalischer bzw. schwach saurer Flotte nach den üblichen Auszieh- und Klotzverfahren oder als Druckfarbstoff klare blaue Färbungen bzw. Drucke mit rotstichiger Nuance auf Baumwolle und Wolle mit sehr guten Nassechtheitseigenschaften.

Beispiel 3

Es wird gemäss Beispiel 1 verfahren, jedoch werden in der zweiten Kondensationsstufe anstelle von 4-β-Thiosulfatoethylsulfonyl-anilin 19 Teile 4-Vinylsulfonylanilin verwendet. Die Aufarbeitung erfolgt analog Beispiel 1 und liefert 131 Teile eines Farbstoffpulvers, welches das Natriumsalz der Verbindung der Formel

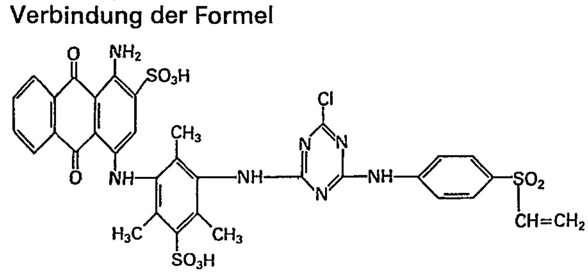

enthält. Dieses liefert nach den für Reaktivfarbstoffe üblichen Färbe- und Druckverfahren auf Wolle und Baumwolle klare, rotstichig blaue Farbtöne mit sehr guten Nassechtheiten.

Beispiele 4 bis 9

Man verfährt analog einer der in den obigen Ausführungsbeispielen beschriebenen Verfahrensweisen, wobei in der zweiten Kondensationsstufe die äquivalente Menge der nachfolgend angegebenen Anilinverbindungen der Formel (4) eingesetzt werden. Die so hergestellten erfindungsgemässen Verbindungen färben beispielsweise Wolle oder Baumwolle ebenfalls in klaren rotstichig blauen Tönen mit sehr guten Nassechtheiten.

| Beispiel | Ausgangsverbindung (2) |
|---|---|
| 4 | 3-β-Sulfatoethylsulfonyl-anilin |
| 5 | 3-Vinylsulfonyl-anilin |
| 6 | 4-Methoxy-anilin-3-β-sulfatoethylsulfon |
| 7 | 2-Methoxy-anilin-5-β-sulfatoethylsulfon |
| 8 | 2-Chlor-anilin-5-β-sulfatoethylsulfon |
| 9 | 2-Methyl-anilin-4-vinylsulfon |

Anwendungsbeispiel 1

15 Teile der erfindungsgemässen Verbindung von Beispiel 1 werden mit 50 Teilen Harnstoff in 200 Teilen heissem Wasser gelöst. Der Lösung werden unter Rühren 400 Teile einer aus 40 Teilen Natriumalginat und 960 Teilen Wasser bestehenden Verdickung sowie 20 Teile Natriumbicarbonat zugegeben. Dann wird die Mischung mit Wasser und Verdickung auf 1000 Teile eingestellt. Mit dieser Druckpaste wird ein Baumwollgewebe bedruckt, das nach dem Trocknen 5 Minuten lang bei 101 bis 103°C gedämpft und sodann mit kaltem, danach mit heissem Wasser gespült, kochend geseift, wieder gespült und getrocknet wird. Man erhält einen rotstichig blauen Druck mit sehr guten Nassechtheitseigenschaften.

Anwendungsbeispiel 2

100 Teile Wollkammzug werden wie üblich vorgewaschen und bei 40°C in 3000 Teile eines wässrigen Färbebades gebracht, welches 1 Teil eines durch Oxethylierung eines $C_{12}$–$C_{14}$-Fettamins mit 5 Mol Ethylenoxid hergestellten Hilfsmittels sowie 2 Teile Ammoniumacetat enthält. Mit Essigsäure wird ein pH-Wert von 4,8 bis 5 eingestellt. In diesem Bad wird das Substrat 5 Minuten lang behandelt. Sodann werden 1,5 Teile der erfindungsgemässen Verbindung von Beispiel 3, in wenig Wasser gelöst, zugegeben. Die Flotte wird innerhalb von 30 Minuten auf Siedetemperatur erhitzt und die Ware 60 Minuten bei dieser Temperatur gefärbt. Anschliessend wird die so erhaltene Färbung in Wasser gespült, angesäuert, wiederum gespült und getrocknet. Die erhaltene Färbung des Kammzuges zeigt einen klaren, kräftigen Blauton von rotstichiger Nuance mit sehr guten Licht- und Nassechtheiten und einwandfreier Egalität.

**Patentansprüche**

1. Verbindungen der allgemeinen Formel (1)

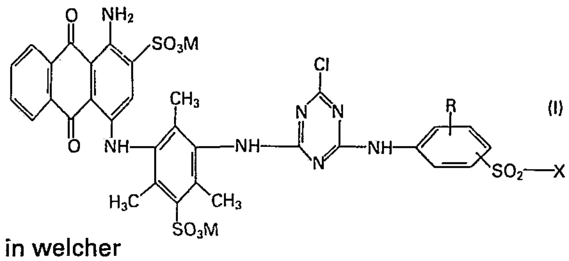

(I)

in welcher

M für ein Wasserstoffatom oder das Äquivalent eines Metalls steht,

R ein Wasserstoffatom, eine Methyl-, Ethyl-, Methoxy-, Ethoxy- oder Carboxygruppe oder ein Halogenatom ist und

X die Vinylgruppe, eine β-Sulfatoethyl-, eine β-Thiosulfatoehtyl- oder die β-Chlorethyl-Gruppe bedeutet.

2. Verbindungen nach Anspruch 1 der dort angegebenen allgemeinen Formel (1), dadurch gekennzeichnet, dass R ein Wasserstoffatom ist, X für die Vinylgruppe oder eine β-Thiosulfatoethyl- oder eine β-Sulfatoethyl-Gruppe steht, die Gruppe der Formel $-SO_2-X$ in p-Stellung zur Aminogruppe an den Benzolkern gebunden ist und M die in Anspruch 1 genannte Bedeutung besitzt.

3. Verfahren zur Herstellung der in Anspruch 1 genannten und definierten Verbindungen der allgemeinen Formel (1), dadurch gekennzeichnet, dass man eine Verbindung der allgemeinen Formel (3)

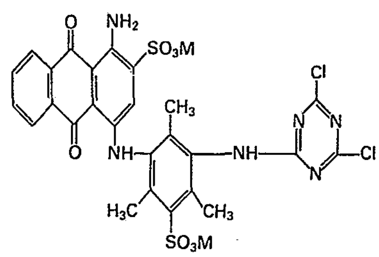

(3)

in welcher M die in Anspruch 1 genannte Bedeutung besitzt, mit einer Aminoverbindung der allgemeinen Formel (4)

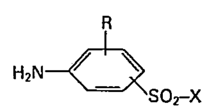

(4)

in welcher R und X die in Anspruch 1 genannten Bedeutungen haben, umsetzt oder dass man eine Anthrachinonverbindung der allgemeinen Formel (2)

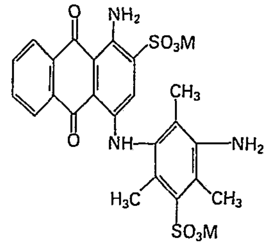

(2)

mit M der in Anspruch 1 genannten Bedeutung

mit einer Dichlortriazinylverbindung der allgemeinen Formel (5)

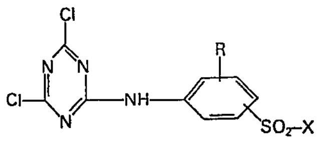

(5)

in welcher R und X die in Anspruch 1 genannten Bedeutungen besitzen, umsetzt.

4. Verwendung der in Anspruch 1 genannten und definierten Verbindungen der allgemeinen Formel (1) zum Färben oder Bedrucken von hydroxy- und/oder carbonamidgruppenhaltigen Materialien, insbesondere Fasern.

5. Materialien aus hydroxy- und/oder carbonamidgruppenhaltigen Fasern, die mit einer Verbindung von Anspruch 1 gefärbt oder bedruckt wurden.

6. Verfahren zum Färben von hydroxy- oder carbonamidgruppenhaltigem Material, bei welchem man einen Farbstoff auf das Material aufbringt oder in das Material einbringt und ihn darauf oder gegebenenfalls darin, gegebenenfalls in der Wärme und/oder in Gegenwart eines säurebindenden Mittels, fixiert, dadurch gekennzeichnet, dass man als Farbstoff eine Verbindung der in Anspruch 1 genannten und definierten allgemeinen Formel (1) einsetzt.

**Claims**

1. Compounds of the general formula (1)

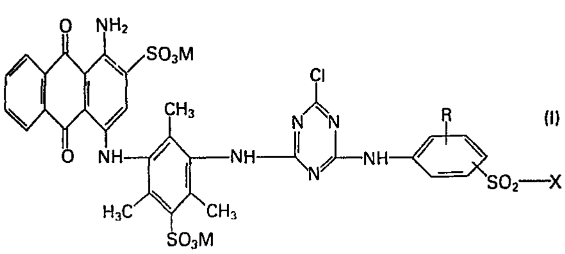

(I)

wherein

M represents a hydrogen atom or the equivalent of a metal,

R is a hydrogen atom, a methyl, ethyl, methoxy, ethoxy or carboxy group or a halogen atom, and

X denotes the vinyl group, a β-sulfatoethyl, a β-thiosulfatoethyl or the β-chloroethyl group.

2. Compounds according to claim 1 of the general formula (1), characterized by that R is a hydrogen atom, X is the vinyl group or a β-thiosulfatoethyl or a β-sulfatoethyl group, the group of the formula $-SO_2-X$ is bonded to the benzene nucleus in the p-position relative to the amino group, and M has the meaning mentioned in claim 1.

3. A process for the manufacture of the compounds of the general formula (1) as mentioned and defined in claim 1, characterized by that a compound of the general formula (3)

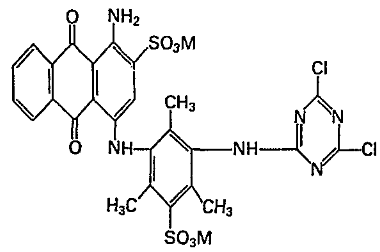

(3)

wherein M has the meaning mentioned in claim 1, is reacted with an amino compound of the general formula (4)

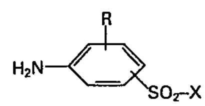

(4)

wherein R and X have the meanings mentioned in claim 1, or that an anthraquinone compound of the general formula (2)

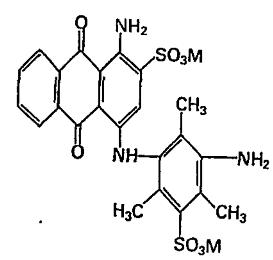

(2)

wherein M has the meaning mentioned in claim 1, is reacted with a dichlorotriazinyl compound of the general formula (5)

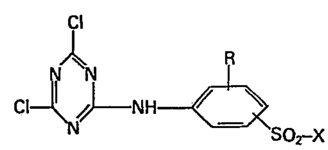

(5)

wherein R and X have the meanings mentioned in claim 1.

4. Use of the compounds of the general formula (1) specified and defined in claim 1, for dyeing or printing materials, in particular fibers, containing hydroxy and/or carbonamide groups.

5. Materials made from fibers containing hydroxy and/or carbonamide groups, which have been dyed or printed with a compound of claim 1.

6. A process for coloring a material containing hydroxy and/or carbonamide groups, wherein a dyestuff is applied to the material or incorporated into the material and fixed thereon or optionally therein, optionally with action of heat and/or in the presence of an acid-binding agent, characterized by that a compound of the general formula (1) specified and defined in claim 1, is used as the dyestuff.

**Revendications**

1. Composés répondant à la formule 1:

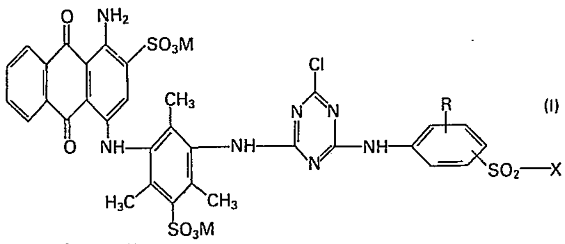

(I)

dans laquelle

M représente un atome d'hydrogène ou l'équivalent d'un métal,

R représente un atome d'hydrogène, un radical méthyle, éthyle, méthoxy, éthoxy ou carboxy ou un atome d'halogène, et

X représente un radical vinyle, un radical sulfato-2 éthyle, un radical thiosulfato-2 éthyle ou un radical chloro-2 éthyle.

2. Composés de formule générale 1 selon la revendication 1, caractérisés en ce que R représente un atome d'hydrogène, X représente un radical vinyle, un radical thiosulfato-2 éthyle ou un radical sulfato-2 éthyle, le radical $-SO_2-X$ étant en position para relativement au radical amino sur le noyau benzénique, et M a la signification donnée à la revendication 1.

3. Procédé de préparation des composés de formule générale 1 selon la revendication 1, procédé caractérisé en ce qu'on fait réagir un composé répondant à la formule générale 3:

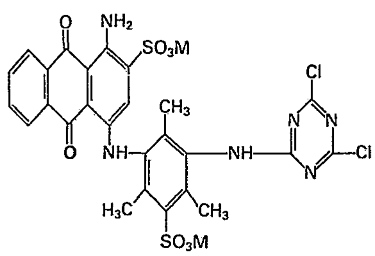

(3)

dans laquelle M a la signification donnée à la revendication 1, avec un composé aminé répondant à la formule générale 4:

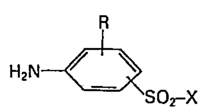

(4)

dans laquelle R et X ont les significations données à la revendication 1, ou on fait réagir un composé anthraquinonique répondant à la formule générale 2:

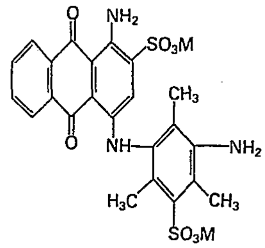

(2)

dans laquelle M a la significations donnée à la revendication 1, avec un composé dichlorotriazinylique répondant à la formule générale 5:

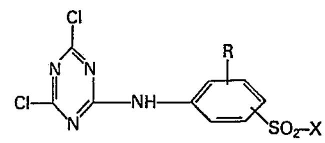

(5)

dans laquelle R et X ont les significations données à la revendication 1.

4. Application des composés de formule générale 1 selon la revendication 1 pour la teinture ou l'impression de matières contenant des radicaux hydroxy et/ou des radicaux carbamoyles, plus particulièrement de matières fibreuses de ce genre.

5. Matières à base de fibres contenant des radicaux hydroxy et/ou des radicaux carbamoyles, qui ont été teintes ou imprimées avec un composé selon la revendication 1.

6. Procédé pour teindre des matières contenant des radicaux hydroxy ou carbamoyles, selon lequel on applique un colorant sur la matière ou on incorpore un colorant dans la matière et on le fixe, sur celle-ci ou éventuellement dans celle-ci, le cas échéant à chaud et/ou en présence d'un accepteur d'acides, procédé caractérisé en ce qu'on utilise, comme colorant, un composé répondant à la formule générale 1 qui a été représentée et définie à la revendication 1.